# EUROPEAN PATENT APPLICATION

(11) **EP 3 225 257 A1**
(43) Date of publication of application: **04.10.2017**
(21) Application number: 16305367.1
(22) Date of filing: 30.03.2016
(51) Int. Cl.: A61K 48/00, A61K 9/51, C12N 15/88

(54) **AMPHIPHILIC MONOMERS BASED NANOVECTORS AND THEIR USE FOR SIRNA DELIVERY**

(71) Applicant: Université de Strasbourg, 67000 Strasbourg (FR); Centre National de la Recherche Scientifique, 75016 Paris (FR)
(72) Inventor: RIPOLL, Manon, 90300 Valdoie (FR); NEUBERG, Patrick, 67300 Schiltigheim (FR); WAGNER, Alain, 67000 Strasbourg (FR); REMY, Jean-Serge, 67470 Schnersheim (FR)
(74) Representative: Pontet Allano & Associes

(57) **Abstract**

Amphiphilic monomer of formula (I)

Li-CΞC-CΞC-L₂-X-Z (I)

wherein
L₁ is CH₃(CH₂)ₘ , with m being an integer from 1 to 24, L₂ is -CH₂(CH₂)ₙ-CONH-, with n being an integer from 1 to 24, X is -(CH₂)ₜ-O-(CH₂CH₂O)ₚ(CH₂)_{t'}-NH-, with t and t' being independently from each other 2 or 3 and p being an integer from 2 to 500 and Z is H or a positively charged group selected from natural or non natural amino acid residues, carboxylated polyamines residues, and peptides comprising from 2 to 5 amino acids, at least one of said amino-acids being an histidine residue, their physiologically acceptable salts and their use in transfection and delivery methods.

## Description

The instant invention concerns amphiphilic monomers, nanovectors based thereon, their process of preparation and their use in transfection methods.

Transfection is the process of deliberately introducing nucleic acids into cells; it is used in particular for non-viral method in eukaryotic cells. There are various methods of introducing foreign DNA into an eukaryotic cell: some rely on physical treatment (electroporation, cell squeezing, nanoparticles, magnetofection), other on chemical materials that are used as carriers. In a commonly accepted way the term transfection is also used for the delivery of small nucleic acids into the cell such as siRNA.

Over the past few years different types of nanoparticles like liposomes, dendrimers, micelles and stable nucleic acid lipid particles (SNALPs) have been explored for the delivery of genes and/or siRNAs into cells. The delivery of siRNAs has revealed particularly interesting since its discovery because it has a great therapeutic potential in all pathologies in which down-regulation of a specific mRNA lead to a beneficial effect paving the way to various clinical trials for the treatment of diseases such as cancers, viral infections and genetic disorders.

WO 2008151150 discloses a composition for siRNA delivery comprising a water soluble degradable crosslinked cationic polymer comprising a recurring backbone polyethylene glycol (PEG) unit, a recurring backbone cationic polyethyleneimine (PEI) unit, and a recurring backbone degradable unit that comprises a side chain lipid group.

When mixed with siRNAs at the optimal ratio the positively charged nanoparticles are taken up by the cells through an endocytosis-mediated process. However, efficient cell uptake is not sufficient since endosomal escape is also a crucial step for achieving gene silencing because the siRNA has to be delivered into the cytoplasm of the cells in order to be able to incorporate into the RNA-induced silencing complexes (RISCs). To achieve the escape from the endosomes, different strategies have been developed among which the use of chemical functions with favorable pKa values such as imidazole groups which induce the so-called "proton sponge" effect (P. Midoux, et al. (2009) Br. J. Pharmacol., 157, 166-178; C. Gonçalves, et al. (2014) Int. J. Pharm., 460, 264-272. Indeed the membrane bound ATPase proton pumps actively translocate protons into endosomes. Buffering of the endosomes through compounds like PEI or imidazole containing compounds leads to a massive proton accumulation followed by passive chloride influx. These events probably cause osmotic swelling and subsequent endosome disruption; this phenomenon is called "proton sponge" effect by J-P. Behr. (P. Neuberg et al. (2014), Adv. Genet., 88, 263-288).

Among novel nanostructured material, some of the inventors reported recently that UV irradiated cationic polydiacetylenic micelles (PDA-micelles) are efficient gene delivery vehicles (E. Morin, et al., (2011) Bioconjug. Chem., 22, 1916-1923). Small 10 nm diameter PDA-micelles were obtained by self-assembly of surfactants composed of a photopolymerizable diacetylenic motif within a C₂₅-hydrophobic chain and a cationic polar head attached with a short hydrophilic trioxatridecane linker. While efficient for DNA delivery, this first generation of vector did however not permit siRNA delivery. In the meantime Doris' group has shown the strong potential of neutral polydiacetylene micelles as cargo for targeted drug delivery. (N. Mackiewicz, et al., (2011), Small, 7, 2786-2792; E. Gravel, et al. (2013), Nanoscale, 5, 1955-1960).

WO2012020337 discloses a polymerized micelle of size inferior to 100 nm for use only in in vivo diagnosis; said polymerized micelle comprises
a diagnostic agent, and an amphiphilic polymer obtainable by the polymerization of an amphiphilic monomer, said monomer comprising:
- a lipophilic chain comprising a polymerizable vinylic or diacetylenic group allowing the polymerization of the micelle, and
- a hydrophilic head comprising a polyoxyethylene or polyoxypropylene chain.

Consequently there is still a need to develop new system for siRNA delivery.

The inventors discovered a mean for optimizing the head group functionality of the PDA-micelles system in order for them to become a promising nanovector for siRNA delivery with up to 80 % of specific inhibition in a gene reporter system.

Consequently a first object of the invention is an amphiphilic monomer of formula (I)

L₁-C≡C-C≡C-L₂-X-Z (I)

wherein
L₁ is CH₃(CH₂)ₘ, with m being an integer from 1 to 24,
L₂ is -CH₂(CH₂)ₙ-CONH-, with n being an integer from 1 to 24,
X is -(CH₂)ₜ-O-(CH₂CH₂O)ₚ(CH₂)_{t'}-NH-, with t and t' being independently from each other 2 or 3 and p being an integer from 2 to 500 and
Z is H or a positively charged group selected from:
   - natural or non natural amino acid residues,
   - carboxylated polyamines residues, and
   - peptides comprising from 2 to 5 amino acids, at least one of said amino-acids being an histidine residue,
and their physiologically acceptable salts.

According to the invention, natural amino acids residues are issued from amino acids selected from the group comprising Histidine, Alanine, Isoleucine, Arginine, Leucine, Asparagine, Lysine, Aspartic acid, Methionine, Cysteine, Phenylalanine, Glutamic acid, Threonine, Glutamine, Tryptophan, Glycine, Valine, Proline, Selenocysteine, Serine, Tyrosine. They may be provided by extraction of natural sources, from commercial sources or chemically synthesized.

According to the invention, non natural amino acid residues are derived from non-proteinogenic amino acids that either occur naturally or are chemically synthesized. As example may be cited β-amino acids (β3 and β2), Homo-amino acids, proline and pyruvic acid derivatives, 3-substituted alanine derivatives, Glycine derivatives, ring-substituted phenylalanine and tyrosine derivatives, linear core amino acids and N-methyl amino acids.

According to the invention, carboxylated polyamines residues like carboxy spermine and homologues thereof are chemically synthesized from amino acid lysine as described in literature.

In an advantageous embodiment of the invention, in the amphiphilic monomer of formula (I), Z is selected from:
- the following natural amino acid residues: 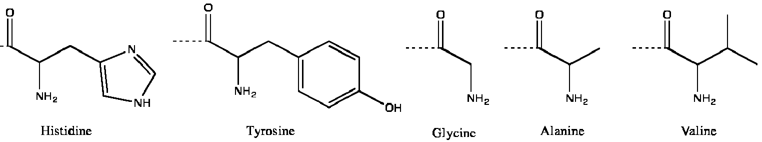
- non natural amino acid residues selected from the group comprising the enantiomers of the above mentioned natural amino acids and their beta and gamma analogues,
- a dipeptide comprising at least one histidine residue,
- a carboxyspermine residue of formula (1), 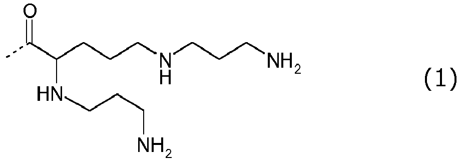
- a -COCH(NH2)(CH2)qA group with q being an integer from 1 to 5 and A being a hydrophilic head being chosen from imidazol-4-yl, imidazol-2-yl, imidazole-1-yl, phenol, 4-methylpiperazine-1-yl, N-substituted cycloamines and analogues thereof.

Another object of the invention is a polymerized micelle comprising a polymer obtainable by the photopolymerization of one monomer of formula (I) or of copolymers of monomers of formula (I) with the proviso that when there is only one monomer, then Z is not H.

According to the invention, when several monomers are photopolymerized to give the polymerized micelles, said monomers may be identical or different.

Another object of the invention is a polymerized fiber comprising a polymer obtainable by the photopolymerization of one monomer of formula (I) or of copolymers of monomers of formula (I) including a polymer obtained from only one monomer wherein Z=H.

According to the invention, when several monomers are photopolymerized to give the polymerized fibers, said monomers may be identical or different.

Another object of the invention is a transfection complex comprising a polymerized micelle or a polymerized fiber as defined above and at least one isolated nucleic acid, micro RNA, small hairpin RNA or mRNA.

As used herein, the term "transfection complex" refers to a mixture of a polymerized micelle or a polymerized fiber according to the invention and a nucleic acid. Transfection is the introduction of foreign matter into cells. Within the context of transfection, "into cells" means transport of the foreign matter across at least one biological membrane. Further transport may occur into cellular compartments or organelles. In contrast, a mere inclusion of the material into endosomes, pinocytic or phagocytic vesicles or lysosomes is not considered a transfection.

In another embodiment of the invention, the isolated nucleic acid is a small interfering RNA.
According to the invention small interfering RNA (siRNA) include RNA having 5 to 50 base pairs, advantageously 15 to 30 base pairs and more advantageously 21 base pairs. RNA may also include micro RNA, small hairpin RNA, mRNA, mixed RNA/DNA molecules or mixed RNA/anticancer drug molecules like cell cycle inhibitors, DNA intercalators, Topoisomerase inhibitors...

According to the invention, the transfection complex may be used in therapy, for example for the treatment of bladder, lung and liver cancers.... Thus another objet of the invention is a method of treating cancer using the transfection complex according to the invention to deliver siRNA into mammalian cancer cells for the treatment of cancer. SiRNA may be administered as a disease treatment. For example siRNA corresponding to all or part of a coding region of a gene that is expressed or overexpressed in a disease state is administered to a patient in need of a treatment.

According to the invention, the transfection complex may be mixed with a pharmaceutically acceptable excipient and optionally micro RNA, small hairpin RNA, mRNA, mixed RNA/DNA molecules or mixed RNA/anticancer drug molecules like cell cycle inhibitors, DNA intercalators, Topoisomerase inhibitors to give a pharmaceutical composition which is also part of the invention. The exact formulation, route of administration and dosage for said pharmaceutical compositions can be chosen by the individual physician in view of the patients conditions. Typically, the dose range of the composition administered to the patient can be from 0.5 to 1000 mg/kg of the patient's body weight, or 1 to 500 mg/kg, or 10 to 500 mg/kg, or 50 to 100 mg/kg of the patient's body weight. The dosage may be a single one or a series of two or more given in the course of one or more days, as needed by the patient.

Another object of the invention is a in vitro or in vivo transfection or delivery method comprising the step of introducing a transfection complex according to the invention into a host cell.

In an advantageous embodiment of the invention, said host cell is an eukaryotic cell including invertebrate and vertebrate cells (among latter mammalian or non-mammalian cells), in particular a mammalian cell (human, monkey, canine, feline, bovine or murine cells) and more particularly a human cell like for example cancer cells.

Another object of the invention is a process for obtaining polymerized micelles or polymerized fibers according to the invention characterized in that it comprises the following steps:
- at least two amphiphilic momomers of formula (I), identical or different, are self-assembled into spherical micelles or fibers,
- the self-assembled spherical micelles or fibers are photopolymerized.

The photopolymerization is realized by strong UV irradiation at 254 nm by devices known by the one skilled in the art and described in the literature. For example irradiation may be realized from 10 min to 4 hours with 48W light bulbs.

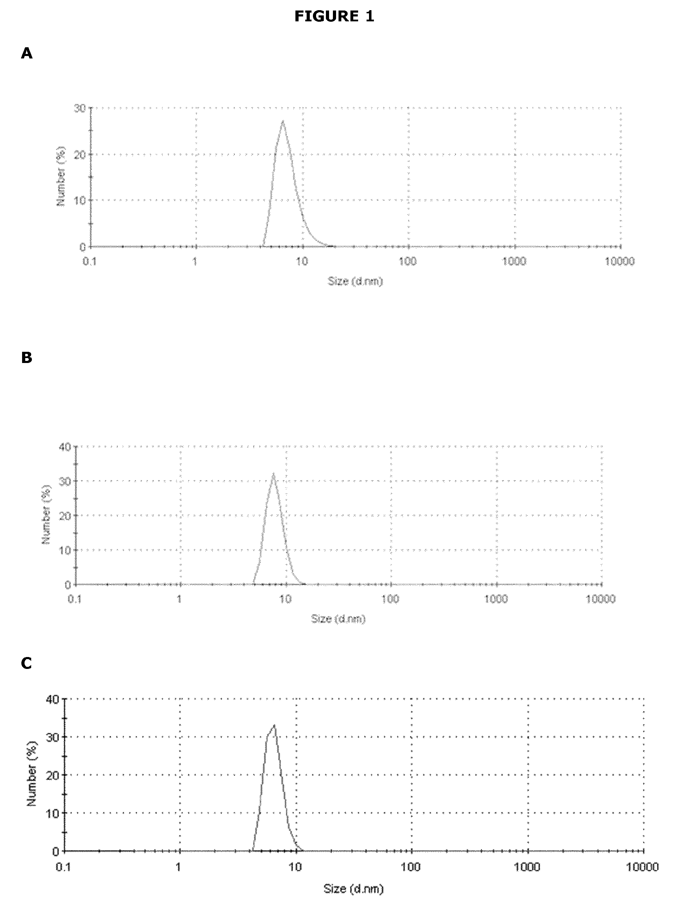

The figures 1 to 10 and examples 1 to 5 illustrate the invention.

In these figures we use following abbreviations:
PM : polymerized Micelle
NPM : non polymerized micelle
Hist-PM : polymerized micelle based upon surfactant molecule 1 bearing histidine headgroups
NH2-PM : polymerized micelle based upon surfactant molecule 2 bearing a free amine
Hist-DPM : dialyzed polymerized micelle based upon surfactant molecule 1 bearing histidine headgroups
Hist-NPM : non polymerized micelle based upon surfactant molecule 1 bearing histidine headgroups
4h-Hist-PM : polymerized micelle based upon surfactant molecule 1 bearing histidine headgroups polymerized for 4 hours
siGL3 : siRNA specifically targeting luciferase reporter gene which sequence is described in 4.1 Material and Methods

Figure 1 illustrates the size distribution by number of polymerized micelles for 4h of photopolymerization according to example 2. (A) NH2-PMs; (B) Hist-PMs; (C) Hist-DPMs corresponding to Hist-PMs dialyzed in 70% ethanol and resuspended in water.

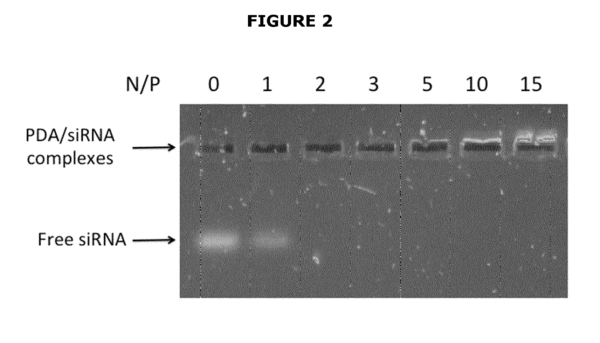

Figure 2 illustrates the siARN siGL3 complexation with the polymerized micelles (4h-Hist-PMs) in low salt conditions at various N/P according to example 3. Lane 0: naked siRNA.

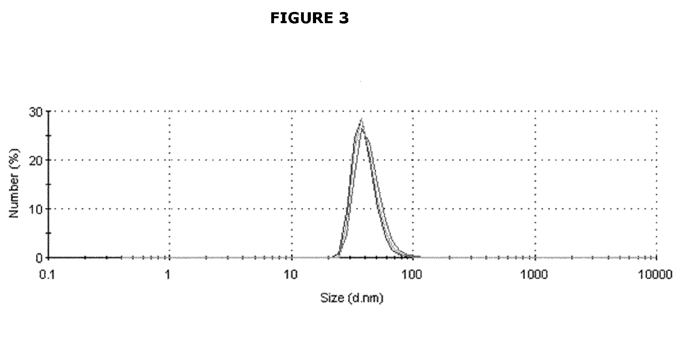

Figure 3 illustrates the size distribution by number of the complexes formed in low salt condition (Hepes Buffer Glucose) between siRNA and Hist-PMs (70 ng/µL siRNA, 20 µg/mL Hist-DPMs) according to example 3.

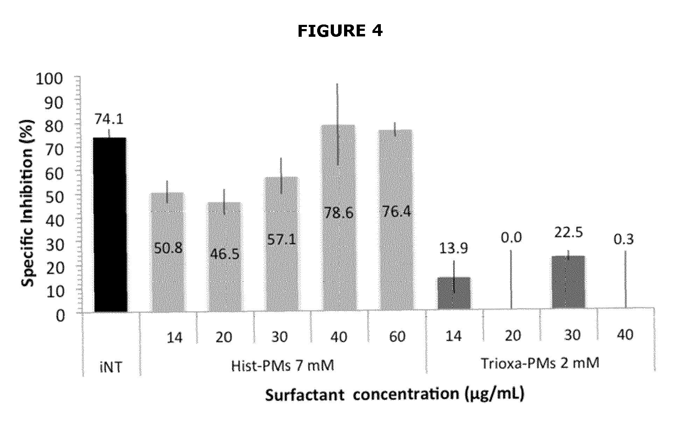

Figure 4 illustrates siRNA delivery experiments using increasing concentrations of polymerized micelles formed with surfactant 1 (Hist-PMs) and with surfactant 2 NH₂-PMs (based on C₂₅diyne-trioxaamine) with 10 nM of siRNA according to example 4. INT (INTERFERin™) is used as positive control.

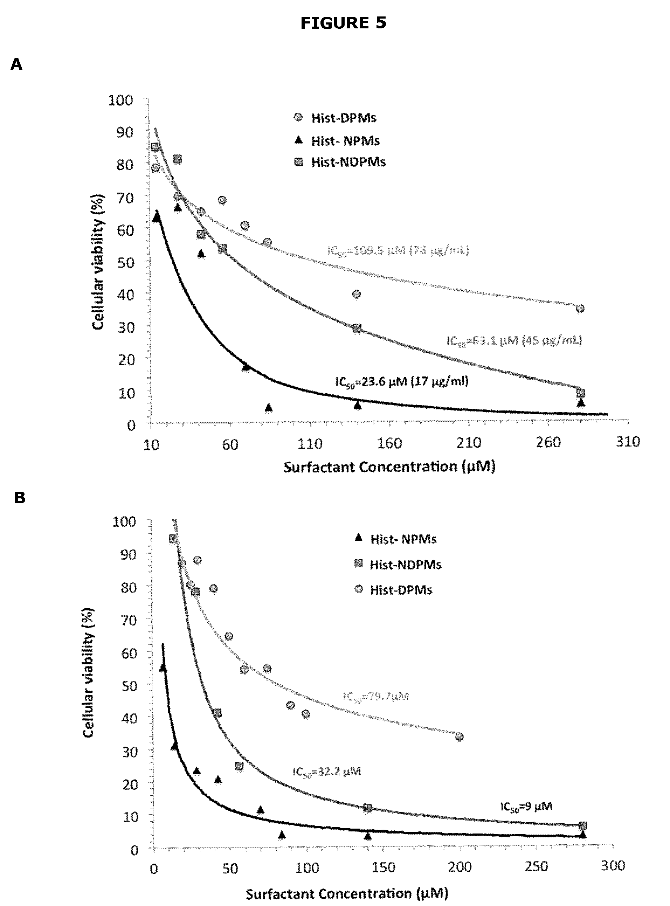

Figure 5: (A) illustrates the results of MTT cytotoxicity assay performed 48h after surfactant deposit on A549-Luc cells according to example 4. Cellular viability is given for non-polymerized micelles (hist-NPMs), 4h-polymerized micelles (hist-NDPMs) and histidine extensively dialysed micelles in 70% ethanol resuspended in water (DPMs in ethanol); (B) illustrates the results of MTT cytotoxicity assay performed 48h after surfactant deposit in HeLa cells according to example 4. Cellular viability is given for non-polymerized micelles (hist-NPMs), 4h-polymerized micelles (Hist-NDPMs) and extensively dialysed micelles (Hist-DPMs).

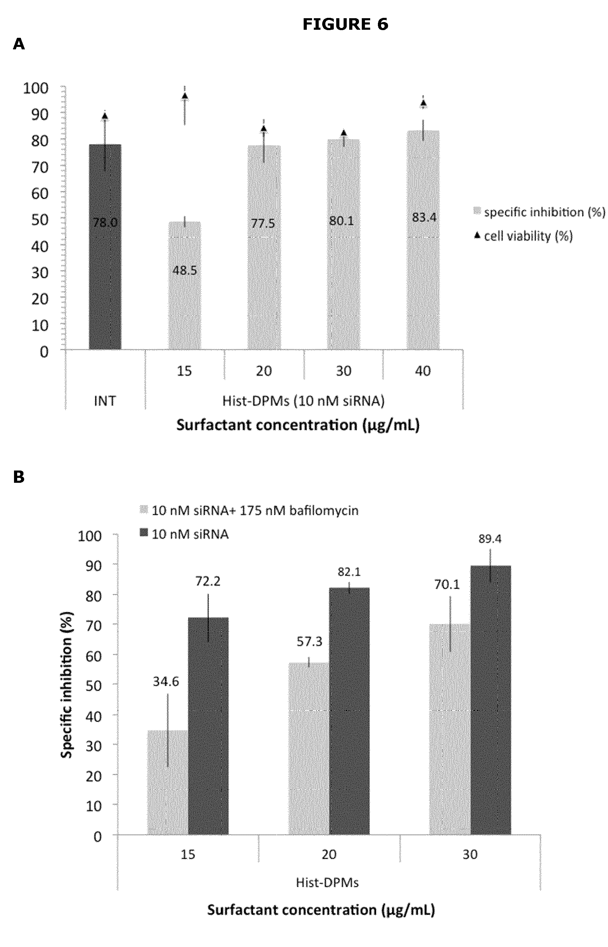

Figure 6 illustrates (A) siRNA delivery experiments performed on A549-luc cells using various concentrations of surfactant 1 with 10 nM siRNA according to example 4. Specific inhibitions compared to A549-luc cells transfected with negative control siRNA after 48h are given for commercial positive control (INT standing for INTERFERin™) (dark grey column) and Hist-DPMs (dialysed polymeric micelles) (light grey columns). Cell viability is measured by total protein assay and represented here compared to non transfected cells (triangles). (B) Effect of bafilomycin A1 on Hist-DPMs transfection activity on A549-luc cells after 48h of transfection. Specific inhibition of luciferase expression as compared to siRNA control are given for hist-DPMs in presence of 10 nM siRNA +/- 175 nM bafilomycin A1.

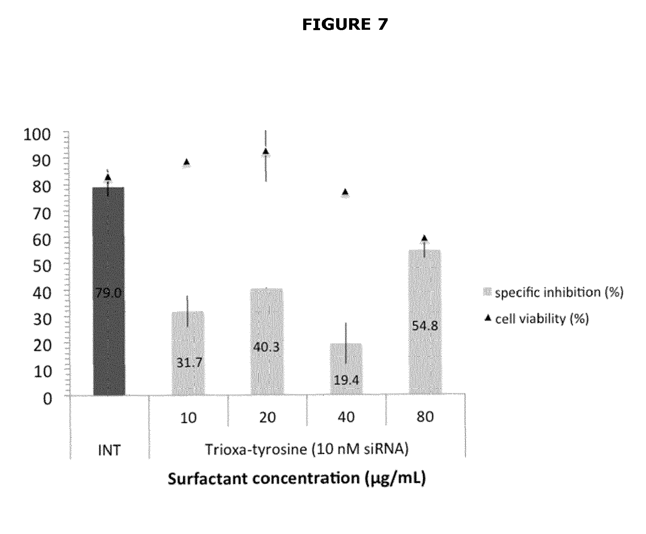

Figure 7 illustrates siRNA delivery experiments performed on A549-luc cells using various concentrations of photopolymerized micelles of compound C25diyne-trioxa-L-Tyrosine (3) with 10 nM siRNA according to example 4. Specific inhibition compared to A549-luc cells transfected with control siRNA after 48h of transfection are given in diagram (light grey columns). A commercial positive control (INT standing for INTERFERin™) is included in experiment (dark grey column). Cell viability is measured by total protein assay and represented here compared to non transfected cells (triangles).

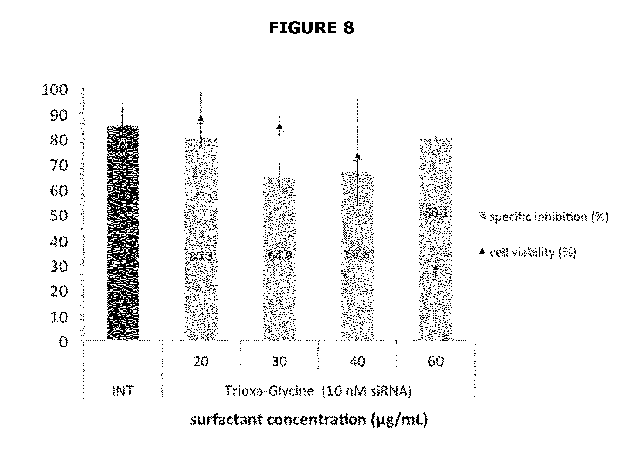

Figure 8 illustrates siRNA delivery experiments performed on A549-luc cells using various concentrations of photopolymerized micelles of compounds C25diyne-trioxa-Glycine (4) with 10 nM siRNA according to example 4. Specific inhibition compared to A549-luc cells transfected with control siRNA after 48h of transfection are given in diagram (light grey columns). A commercial positive control (INT standing for INTERFERin™) is included in experiment (dark grey column). Cell viability is measured by total protein assay and represented here compared to non transfected cells (triangles).

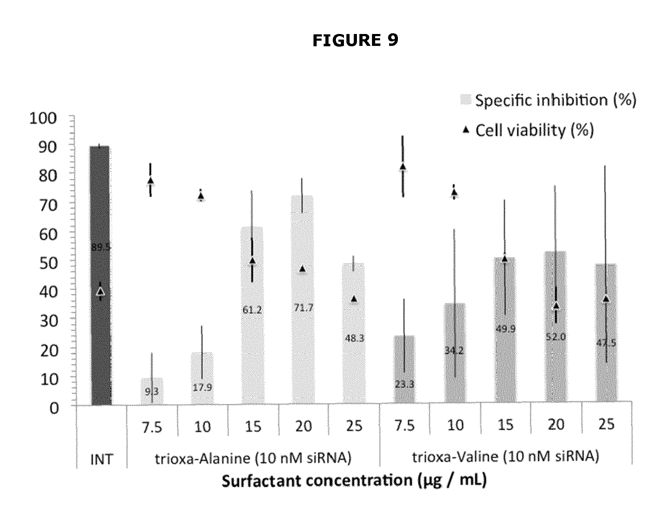

Figure 9 illustrates siRNA delivery experiments performed on A549-luc cells using various concentrations of photopolymerized micelles of compounds C₂₅diyne-trioxa-L-Alanine (5) and C₂₅diyne-trioxa-L-Valine (6) with 10 nM siRNA according to example 4. Specific inhibition compared to A549-luc cells transfected with control siRNA after 48h of transfection are given in diagram (light grey columns). A commercial positive control (INT standing for INTERFERin™) is included in experiment (dark grey column). Cell viability is measured by total protein assay and represented here compared to non transfected cells (triangles).

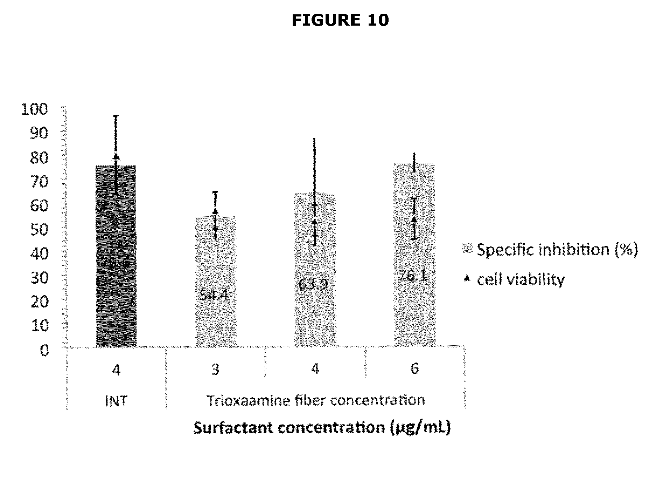

Figure 10 illustrates siRNA delivery experiments performed on A549-luc cells using various concentrations of photopolymerized nanofibers according to example 6 obtained from compound 2 C₂₅diyne-trioxaamine with 10 nM siRNA according to example 4. Specific inhibition compared to A549-luc cells transfected with control siRNA after 48h of transfection are given in diagram (light grey columns). A commercial positive control (INT standing for INTERFERin™) is included in experiment (dark grey column). Cell viability is measured by total protein assay and compared to the viability of non transfected cells negative controls (triangles).

### EXAMPLE 1: Synthesis

### 1.1 : Synthesis of C₂₅diynetrioxaamine (compound 2)

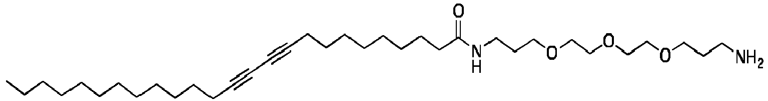

To a solution of 10,12-pentacosediynoic acid (1 eq, 10 g, 26.7 mmol) and N-hydrosuccinimide (1.5 eq, 4.61 g, 40 mmol) in dichloromethane were added 1-ethyl-(3-dimethylaminopropyl)carbodiimide hydrochloride (1.5 eq, 7.68 g, 40 mmol) and N,N-diisopropylethylamine (1.5 eq, 5.18 g, 6.5 mL, 40 mmol). The mixture was stirred overnight at room temperature out of direct light. After evaporation of the solvents the crude product was dissolved in ethyl acetate and extracted with water. The organic phase was dried over anhydrous sodium sulfate, filtered off and evaporated. The obtained white solid (activated ester) was dissolved in dry tetrahydrofuran and 4,7,10-trioxa-1,13-tridecanediamine (2.5 eq, 6 g, 26.5 mmol) was added drop by drop. The reaction mixture was stirred at room temperature overnight. The solvent was evaporated under reduced pressure and the crude product was purified by column chromatography using dichloromethane/methanol/NH₄OH 9:0.9:0.1 as eluent affording compound **2**. (4.25 g, 7.4 mmol, 70%).
**Yield:** 70%
**¹H NMR** (400 MHz, MeOD) δ 3.69-3.63 (m, 8H), 3.61-3.58 (m, 2H), 3.51 (t, 2H), 3.23 (t, 2H), 3.10 (t, 2H), 2.22 (t, 4H), 2.16 (t, 2H), 1.93 (q, 2H), 1.76 (q, 2H), 1.63-1.59 (m, 2H), 1.54-1.47 (m, 4H), 1.44-1.37 (m, 4H), 1.37-1.27 (m, 22H, alkyne chain), 0.90 (t, *J*=7 Hz, 3H) ppm.
**¹³C NMR** (101 MHz, MeOD) δ 174.9, 76.6, 76.5, 70.2, 69.9, 69.8, 69.1, 68.5, 65.0, 38.7, 36.4, 35.8, 31.7, 29.4-28.1, 25.6, 22.3, 18.3, 13.1 ppm. **LCMS:** m/z 577.5 [M+H]⁺

### 1.2. Synthesis of C₂₅diynetrioxa-L-Histidine (compound 1)

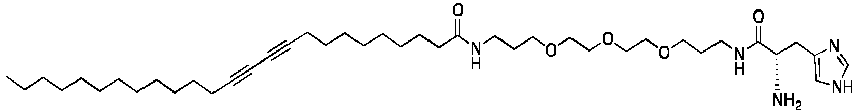

To a solution of compound **2** (1 eq, 510 mg, 0.884 mmol) in dried dichloromethane, *N*α-(tert-butoxycarbonyl)-L-histidine (1.2 eq, 271 mg, 1.06 mmol), 1-ethyl-(3-dimethylaminopropyl)carbodiimide hydrochloride (1.5 eq, 254 mg, 1.33 mmol) and N,N-diisopropylethylamine (15 eq, 0.2 mL, 1.33 mmol) were added. The reaction mixture was stirred overnight under argon atmosphere at room temperature. Then the solvent was evaporated under reduced pressure and the crude product was purified by column chromatography using dichloromethane/methanol/NH₄OH 9/0.9/0.1 as eluent affording the C₂₅diyenetrioxa-*N*α-(tert-butoxycarbonyl)-L-histidine (620 mg, 86%).

To a solution of the last compound in dried dichloromethane, trifluroroacetic acid (50 eq, 2.8 mL, 38.1 mmol) was added at 0°C and the reaction mixture was stirred under argon atmosphere at room temperature overnight. The solvent was evaporated under reduced pressure and the crude product was purified by column chromatography using dichloromethane/methanol/NH₄OH 9/0.9/0.1 as eluent affording desired product **1** (360 mg, 66%).
**Yield:** 66%
**¹H NMR** (400 MHz, MeOD) δ 8.97 (s, 1H), 7.52 (s, 1H), 4.17 (t, *J*= 7 Hz, 1H), 3.65-3.55 (m, 8H), 3.53-3.44 (m, 4H), 3.35-3.22 (m, 6H), 2.24 (t, *J*=6.₅ Hz, 4H), 2.18 (t, *J*=7.5 Hz, 2H), 1.77-1.69 (m, 4H), 1.64-1.57 (m, 2H), 1.54-1.47 (m, 4H), 1.43-1.28 (m, 26H, alkyn chain), 0.9 (t, *J*=7 Hz, 3H) ppm.
**¹³C NMR** (101 MHz, MeOD) δ 174.9, 161.6, 135.5, 132.6, 118.4, 115.9, 115.4, 76.6, 76.5, 70.1, 69.9, 69.8, 68.5, 68.3, 65.0, 53.5 38.7, 36.6 36.4, 35.8, 31.7, 29.4-28.1, 25.6, 22.3, 18.3, 13.1 ppm.
**HRMS (ESI)**: m/z 714.554 ([M+H]⁺, calcd. for C₄₁H₇₂N₅O₅ 714.554)

### 1.3 Synthesis of C₂₅diynetrioxa-L-Tyrosine (compound 3)

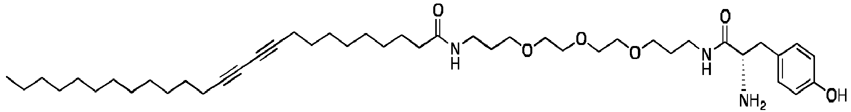

To a solution of C₂₅diynetrioxaamine **2** (1 eq, 150 mg, 0.404 mmol) in dried dichloromethane (DCM), Boc-L-tyrosine hydroxysuccinimide (1.5 eq, 357 mg, 0,619 mmol) and Et₃N (2 eq, 0.1 mL, 0,809 mmol) were added. The reaction mixture was stirred overnight at room temperature under argon atmosphere. The reaction mixture was concentrated under reduced pressure and the resulting mixture was extracted with DCM and finally purified by column chromatography using DCM/MeOH/NH₄OH 9/0.9/0.1 as eluent affording the C₂₅diyenetrioxa-*N*α-(tert-butoxycarbonyl)-L-histidine (220 mg, 66%).

To a solution of the last compound in dried dichloromethane, TFA (50 eq, 1 mL, 14 mmol) was added at 0°C and the reaction mixture was stirred under argon atmosphere at room temperature overnight. The solvent was evaporated under reduced pressure and the crude product was purified by column chromatography using DCM/MeOH/NH₄OH 9/0.9/0.1 as eluent affording desired product **3** (170 mg, 88%).
**Yield:** 88%
**¹H NMR** (400 MHz, MeOD) δ 7.04 (d, *J*=7.9 Hz, 2H), 7.52 (d, *J*=7.9 Hz, 2H), 3.66 (t, *J*=7.6 Hz, 1H), 3.63-3.48 (m, 10H), 3.40-3.13 (m, 6H), 2.98-2.82 (m, 2H), 2.24 (t, *J*=6.4 Hz, 4H), 2.17 (t, *J*=7.4 Hz, 2H), 1.94-1.71 (m, 2H), 1.70-1.64 (m, 2H), 1.64-1.57 (m, 2H), 1.54-1.44 (m, 4H), 1.43-1.25 (m, 26H, alkyne chain), 0.9 (t, *J*=7 Hz, 3H) ppm.
**¹³C NMR** (101 MHz, MeOD) δ 176.8, 171.9, 156.4, 130.1, 126.4, 115.2, 76.5, 70.2-68.4, 65.1, 55.7, 38.6, 36.5-35.8, 31.7-28.1, 25.6, 22.4, 18.3 and 13.01 ppm.
**HRMS (ESI)**: m/z 762.538 ([M+Na]⁺, calcd. for C₄₄H₇₃N₃NaO₆ 762.538)

### 1.4 Synthesis of C₂₅diynetrioxa-Glycine (compound 4)

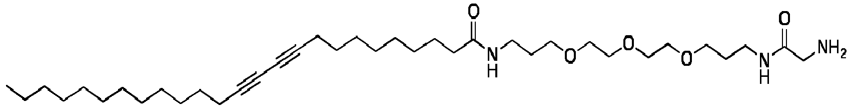

To a solution of natural Fmoc-amino-acids (1.3 eq) in anhydrous DMF (5 mL) were added DIEA (1.3 eq), HATU (1.3 eq) and the C₂₅diynetrioxaamine **2** (0.17 mmol, 1 eq) which was previously solubilized in anhydrous DCM (2 mL). The reaction mixture was stirred at room temperature overnight under argon atmosphere and the mixture was concentrated under reduced pressure. The crude product was then quickly purified by column chromatography using DCM/MeOH 9/1 as eluent affording the corresponding Fmoc-coupling compound.

To a solution of the last compound in DMF (3mL) was added piperidine (1mL). The reaction mixture was stirred for 2 hours under argon atmosphere. The mixture was concentrated under reduced pressure and the crude product was purified by column chromatography using a reversed-phase C18 column (eluent: MeOH- water containing 0.05% TFA) to afford the corresponding deprotected compounds.
**Yield:** 49%
**¹H NMR** (400 MHz, CDCl₃) δ 3.67-3.53 (m, 12H), 3.42-3.31 (m, 6H), 2.23 (t, *J*=7.6, 4H), 2.14 (t, *J*=7.2, 2H), 1.84-1.74 (m, 4H), 1.64-1.59 (m, 2H), 1.55-1.46 (m, 4H), 1.40-1.23 (m, 26H, alkyne chain), 0.88 (t, *J*=6.8 Hz, 3H) ppm.
**¹³C NMR** (101 MHz, CDCl₃) δ 173.5, 77.6, 70.7-69.8, 65.3, 44.5, 37.5-36.7, 31.9, 29.7-28.3, 25.8, 22.7, 19.2, 14.1 ppm.
**LCMS (ESI+):** m/z 634.70 [M+H]⁺

### 1.5 Synthesis of C₂₅diynetrioxa-L-Alanine (compound 5)

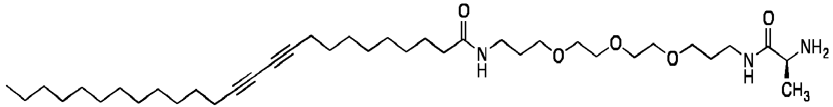

A solution of C₂₅diynetrioxaamine **2** (1 eq, 295.5 mg, 0.5 mmol) in dried dichloromethane (30 mL) was stirred for 10 minutes over molecular sieve. Boc-L-alanine hydroxysuccinimide (1.2 eq, 284.3 mg, 0.6 mmol) and Et₃N (1 eq, 0.07 mL, 0.5 mmol) were added. The reaction mixture was stirred for 6 days at room temperature. The reaction mixture was concentrated under reduced pressure and adsorbed on cellulose for chromatography. Compound was purified by silica gel column chromatography eluting with dichloromethane then with DCM/MeOH/NH₄OH mixtures (step gradients from 95/5/0.5 to 85/15/1.5) affording the C₂₅diyenetrioxa-*N*α-(tert-butoxycarbonyl)-L-alanine (293 mg, 77%).

Isolated N-Boc-protected compound (1 eq, 280 mg, 0.37 mmol) was cooled to 5°C and stirred with excess TFA (5 mL, 183 eq, 67 mmol) in an ice/water bath for 3 hours. The TFA is evaporated under reduced pressure in a rotary evaporator. The crude product was purified by silica gel column chromatography eluting with DCM/MeOH/NH₄OH 90/10/1 affording deprotected product **5** C₂₅diynetrioxa-Alanine (170 mg, 70% yield).
**Yield:** 70%
**¹H NMR** (400 MHz, CDCl₃) δ 7.55-7.42 (m, 1 H), 7.41-7.38 (m, 1 H), 6.35-6.25 (m, 1 H), 3.68-3.57 (m, 8 H), 3.55 (s, 4 H), 3.46 (d, *J*=6.78 Hz, 1 H), 3.40 - 3.30 (m, 4 H), 2.23 (t, *J*=6.90 Hz, 4 H), 2.13 (t, *J*=7.65 Hz, 2 H), 1.83-1.73 (m, 4 H), 1.69-1.64 (m, 3 H), 1.63-1.55 (m, 2 H), 1.55-1.46 (m, 4 H), 1.40-1.35 (m, 3 H), 1.32 (d, *J*=7.03 Hz, 3 H), 1.30-1.21 (m, 23 H), 0.87 (t, *J*=6.78 Hz, 3 H) ppm.
**¹³C NMR** (101 MHz, CDCl₃) δ 175.69, 173.11, 77.62, 77.54, 70.56, 70.03, 70.21, 65.33, 65.26, 50.86, 37.76, 37.23, 36.83, 31.93, 29.66-28.34, 25.78, 22.70,21.81, 19.23, 19.21, 14.13 ppm.

### 1.6 Synthesis of C₂₅diynetrioxa-L-Valine (compound 6)

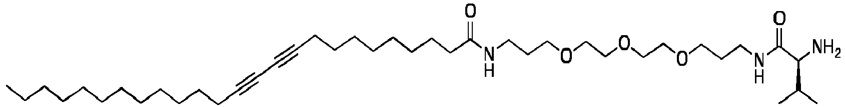

A solution of C₂₅diynetrioxaamine **2** (1 eq, 295.5 mg, 0.5 mmol) in dried dichloromethane (30 mL) was stirred for 10 minutes over molecular sieve. Boc-L-valine hydroxysuccinimide (1.2 eq, 284.3 mg, 0.6 mmol) and Et₃N (1 eq, 0.07 mL, 0,5 mmol) were added. The reaction mixture was stirred for 6 days at room temperature. The reaction mixture was concentrated under reduced pressure and adsorbed on cellulose for chromatography. Compound was purified by silica gel column chromatography eluting with dichloromethane then with DCM/MeOH/NH₄OH mixtures (step gradients from 95/5/0.5 to 85/15/1.5) affording C₂₅diyenetrioxa-*N*α-(tert-butoxycarbonyl)-L-valine (513 mg, 77% pure).

Isolated N-Boc-protected compound (1 eq, 500 mg, 0.49 mmol) was cooled to 5°C and stirred with excess TFA (5 mL, 183 eq, 67 mmol) in an ice/water bath for 3 hours. The TFA is evaporated under reduced pressure in a rotary evaporator. The crude product was purified by silica gel column chromatography eluting with DCM/MeOH/NH₄OH 90/10/1 affording deprotected compound **6** C₂₅diynetrioxa-Valine (260 mg, 0.38 mmol, 77% yield)
**Yield:** 77%
**¹H NMR** (400 MHz, CDCl₃) δ 7.53-7.45 (m, 1 H), 6.34-6.26 (m, 1 H), 3.68-3.52 (m, 12 H), 3.41-3.31 (m, 4 H), 3.23-3.17 (m, 1 H), 2.24 (t, *J*=6.90 Hz, 4 H), 2.14 (t, *J*=7.65 Hz, 2 H), 1.88-1.69 (m, 4 H), 1.68-1.56 (m, 4 H), 1.56-1.48 (m, 4 H), 1.41-1.33 (m, 4 H), 1.32-1.23 (m, 22 H), 0.98 (d, *J*=6.78 Hz, 3 H), 0.88 (t, *J*=6.65 Hz, 3 H), 0.83 (d, *J*=7.03 Hz, 3 H) ppm.
**¹³C NMR** (101 MHz, CDCl₃) δ ppm 173.10, 77.61, 70.54, 70.14, 70.16, 65.33, 65.26, 60.35, 37.78, 37.02, 36.82, 31.93, 31.00, 29.66-28.98, 25.78, 22.69, 19.74, 19.23, 19.21, 16.23, 14.12 ppm.

### EXAMPLE 2: Formation, polymerization and dialysis of the micelles

### 2.1. Material and methods

### 2.1.1. Formation, polymerization and dialysis of the micelles

The formation of micelles was performed by solubilizing 5 mg of amphiphile from 1 to 6 obtained in example 1 into a solution of HCl 0.1 N and deionized water (1/5). The solvent was evaporated under reduced pressure until a film formation. The film was then solubilized in 1 mL of deionized water and sonicated during 30 min (80 W, 25°C) to obtain non-polymerized micelles (NPMs) from compound 1 to 6. The solution was polymerized by UV-irradiation during 4 hours at 254 nm and 48 W in 1 mL quartz cuvettes into a Cross-Linker Bio-Link 254 (Fisher Bioblock) to obtain polymerized micelles (PMs) from compound 1 to 6.

The dialyses of compound 1 was performed with 2,000 MWCO dialysis cassettes (Thermo Fisher) with 70% ethanol and then resuspended in water for 4 days.

### 2.1.2. Size measurements by Dynamic Light Scattering (DLS)

The hydrodynamic diameters of micelles were determined by the Zetasizer Nano ZS system (Malvern Instruments) with the following specifications: sampling time = 55 s; refractive index of water; temperature = 25°C. The values correspond to average size ± standard deviation of three runs.

### 2.2. Results

The hydrodynamic diameters of the polymerized micelles, NH₂-PMs, Hist-PMs and Hist-PMs after dialysis (Hist-DPMs) are given in figure 1. This results showed that all the particules are spherical objects of 10 nm diameter independent of the time of polymerization.

### EXAMPLE 3 : Complexation of siRNA

### 3.1. Material and methods

Complexes were prepared by mixing 300 ng of siRNA (siGL3) with increasing amount of Hist-PMs in Hepes buffered glucose (pH 7.5). The resulting complexes have different N/P ratio, which are defined by the amount of positive charges of the micelles (N) and the number of phosphate groups of the siRNA (P). The complexes were incubated for 1 hour at 25 °C. All the samples were electrophoresed (80 V, 30 min) in a 1.3 % (w/v) agarose gel, stained with sybersafe and visualized on an UV transilluminator using a Gel Documentation System (Bio-Rad).

### 3.2. Results

They are given in figures 2 and 3.

Complete complexation of the nucleic acids took place at low N/P ratio (=2) (Figure 2).

The size of the particles was measured by DLS according to example 2 and the results showed that the siRNA-micelles complexes remained smaller than 100 nm (Figure 3).

### EXAMPLE 4 : siRNA delivery

### 4.1. Material and methods

### 4.1.1. Cell culture

The A549-luc cells that stably express the reporter gene luciferase GL3 (1-2 x 10¹⁰ RLU/mg protein) were established by transfecting human lung carcinoma A549 cells (CCL-185; ATCC) with pGL3-Luc plasmid (Clontech, Mountain View, CA, USA) using jetPEI™ as delivery system (Polyplus-Transfection, Illkirch, France). Cells were grown in RPMI 1640 medium (Eurobio, Les Ulis, France) supplemented with 10% Fetal Bovine Serum (Eurobio, Les Ulis, France), 1% antibiotic solution (pencillin-streptomycin, Gibco-invitrogen) and maintained under 0.8 µg/mL G418 selection (Promega, Madison, WI, USA) at 37 °C in a 5% CO₂ humidified atmosphere.

Human cervical cancer cells (HeLa) were cultured in Dulbecco's modified Eagle medium (DMEM, high glucose, Gibco-Invitrogen) supplemented with 10% Fetal Bovin Serum (Eurobio), 1% antibiotic solution (penicillin-streptomycin, Gibco-invitrogen) in a 5% CO₂ humidified atmosphere at 37°C.

### 4.1.2. siRNA delivery

We used specific siRNA synthesized (synthesized, RP-HPLC purified and annealed by Eurogentec) of following sequences for composing single strands
Sens strand : GL3-ssSIRNA : 5'- CUU ACG CUG AGU ACU UCG A dTdT-3' (SEQ ID NO 1)
Antisens strand GL3-asSIRNA : 5'-U CGA AGU ACU CAG CGU AAG dTdT-3' (SEQ ID NO 2)

We used non coding control siRNA (synthesized, RP-HPLC purified and annealed by Eurogentec) of following sequences for composing single strands
Sens strand: 5' -CGU ACG CGG AAU ACU UCG AdTdT -3'(SEQ ID NO 3) Antisens strand: 5'-U CGA AGU AUU CCG CGU ACG dTdT-3' (SEQ ID NO 4)

Twenty-four hours prior to transfection, 2.5 x 10⁴ A549-luc cells were seeded per well in 24-well tissue culture plates. Complexes were formed between target or control siRNA (siGL3 or siCTL respectively) and the cationic micelle by adding different amounts of surfactant on the siRNA pre-diluted in Hepes Buffer Glucose. Samples were mixed and incubated one hour at room temperature to favor association between siRNA and the cationic micelles. 100 µL of the complexes were added onto the cells in absence of serum in 500 µL RPMI 1640 medium. After 4 h of incubation, 10 µL of fetal bovine serum (Eurobio Les Ulis, France) were added to obtain 10% serum concentration. For positive control, cells were transfected with 10 nM of siGL3 and siCTL vectorized with 2 µL of INTERFERin™ (Polyplus-Transfection, Illkirch, France) according to the supplier recommendations. All experiments were done in triplicate.

### 4.1.3. Quantification of the luciferase gene silencing

Luciferase gene expression was determined 48 h after delivery with a Luciferase Assay System (Promega, Charbonnières, France) according to manufacturer's instructions. The luminescence was quantified using 2 µL of cell lysate with a Centro LB luminometer (Berthold, Thoiry, France). Protein concentration in cell lysate was measured by using a BCA assay kit (Interchim, Montluçon, France), according to manufacturer's instructions. After expressing the luciferase activity as relative light units integrated over 10 s per mg of cell protein (RLU/mg), the luciferase gene silencing was calculated relative to the luciferase activity of non-transfected A549-luc cells.

### 4.1.4 Cytotoxicity assay

Cytotoxicity of compounds was evaluated on A549-luc and HeLa cells. Twenty-four hours prior to the assay, 1.25x 10⁴ HeLa or A549-luc cells were seeded per well in 96-well tissue culture plates. Micelles were then added to cells in serum-free conditions for 4h and 10 µL of serum was then added. Cell viability was measured after 48h by quantifying the mitochondrial succinate dehydrogenase using an MTT assay (Invitrogen). The blue precipitate formed was solubilized by DMSO and quantified by spectrophotometry at 570 nm on a Multiskan FC (Thermo scientific).

### 4.1.5 Transfection in the presence of bafilomycin A1

To determine the role of the histidine group in enhancing the escape of the siRNA from the endosomes, A549-luc cells were transfected with Hist-DPMs and treated with the vacuolar type H⁺-ATPases inhibitor, bafilomycin A1 (Sigma-Aldrich) (BafA1, 175 nM final concentration in the culture medium).

Cells were seeded at a density of 2.5 × 10⁴ cells cm⁻² (in 500 µl of RPMI) in 24-well cell culture plates and incubated for 24 h at 37 °C in a 5% CO₂ atmosphere. siRNA-Hist-DPMs complexes were prepared at various N/P molar ratio, as described in example 2. 1.5 µL of BafA1 solution (40 µM) were added to each well 30 min prior the complex addition in absence of serum and then the complexes were added.
The incubation period with BafA1 lasted for 3 h in absence of serum, after which the medium was refreshed and replaced by complete medium. Luciferase gene expression was determined 48 h after delivery as described in example 4.1.3.

### 4.2. Results

They are given in figures 4 to 10.

The nature of the headgroup plays a crucial role in siRNA delivery. Indeed, 80 % of specific inhibition was reached with 40 µg/ml of compound 1 whereas the NH₂-system 2 only poorly performed in these experiments with a maximum inhibition of 25 % (Figure 4).

The results on the A549-Luc showed that the non-polymerized micelles (IC50=23.6 µM) were almost 3-times more toxic than the polymerized ones (IC50=63.1 µM) (Figure 5A), which confirm the interest of the polymerization. The dialysed 4h-polymerized micelles (Hist-DPMs) in 70 % ethanol resuspended in water presented the lowest toxicity (IC50= 109.5 µM) compared to the non-dialysed one (IC50=63.1 µM).

A similar reduced toxicity of the polymerized micelles was also observed when using human cervical cancer cells (HeLa; Figure 5B).

The siRNA delivery potential of the Hist-DPMs were then evaluated on A549-luc cells again to show if the reduced presence of monomers impacts the biological activity. The efficiency of inhibition was calculated relative to the non-transfected A549-luc cells and compared to a siRNA control (siCTL). The efficiency of the optimized nanovector obtained by dialysis is shown in figure 6A. Up to 80% of inhibition of the luciferase gene expression was obtained with 20 µg/mL of Hist-DPMs. In comparison to the non-dialysed system, the dialysed sample permits to reach the same maximal specific inhibition but by adding less surfactant.

Finally, to gain insight in the biological mechanism of siRNA release in the cytoplasm, we evaluated the effect In presence of bafilomycin A1 which is an antibiotic that selectively inhibits vacuolar type H+ ATPase (V-ATPase), said V-ATPases causing acidification of the internal space of several organelles of the vacuolar system including endosomal compartments. As shown on figure 6B, we observed a significantly lower specific inhibition when the transfection was realised in presence of bafilomycin A1. These experiments evidence that the acidification of the endosomes plays an important role in the mechanism of action of the histidine containing PDAs. Protonation of the imidazole group probably allows a proton sponge like effect that ultimately results in an enhanced cytosolic delivery of the siRNAs.

In figures 7, 8 and 9 we examplify the claimed invention further by showing the delivery potential of a diverse set of molecules of general formula (I). Notably we show that the histidine moiety can be substituted by other amine acid residues, such as glycine, tyrosine, alanine or valine. Obtained molecules show up to 80 % silencing activity in siRNA delivery experiments.

In figure 10 we present a particular fiber form of C25diyne-trioxaamine **2** that shows great delivery potential for siRNA. The preparation of this particular form of surfactants of claim (I) is described in detail in example 6.

### EXAMPLE 5 Procedure for preparing PDA nanofibers as examplified by molecule C25diyne-trioxa-L-Histidine (compound 1)

### 5.1. Preparation of an acidic solution of histidine-trioxaamine (micellar solution)

10mg of C₂₅diynetrioxa-L-Histidine are dissolved in an ethanolic solution of concentrated hydrochloric acid and ethanol (0.1 ml HCl 36% and 0,9 ml ethanol) and evaporated to dryness. The film is taken up in pure water (1ml), and sonicated to form a micellar solution by sonication in a sonication bath.

### 5.2. Precipitation in an alkaline aqueous solution

Introduction of this micellar solution into a aqueous solution of Trizbase (MW = 121.1 ; 3 mmoles ; 363 mg in 10 ml).

The solution is placed at 5°C for 1 hour leading to formation of a nano-emulsion.

### 5.3. Photo-polymerization

Introduction of the previously formed nanoemulsion into 2 Petri dishes (35 mm in diameter)
Irradiation at 254 nm during 10 minutes in an UV-crosslinker (six 8W light tubes ; Biolink BLX-E254 Vilbert Lourmat)

Samples turn red and opaque with fine aggregates.

### 5.4. pH neutralization and dissociation of aggregates

Photopolymerized solution of histidine-trioxaamine is neutralized by addition of concentrated hydrochloric acid (3 mmoles).

The initial precipitate dissociates and becomes transparent and with an intense red colour. These red solutions contain the photopolymerized C₂₅diynetrioxa-L-Histidine in nanofiber form.

### EXAMPLE 6 : Procedure for preparing PDA nanofibers as examplified by molecule C25diyne-trioxaamine (compound 2)

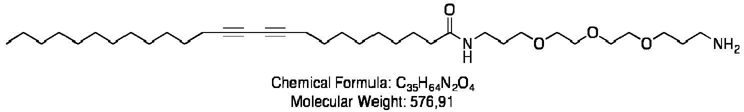

### 6.1. Preparation of a concentrated ethanolic solution of DA-trioxaamine

Dissolution of C₂₅diynetrioxaamine in ethanol at 50°C in a sonication bath. (60 mg dissolved in 6 ml ethanol ; 0.104 mmoles)
An aqueous solutions of ethanol is prepared (42 ml pure water with 12ml ethanol)

### 6.2. Microprecipitation

Injection of the previously formed concentrated C₂₅diynetrioxaamine solution into an aqueous ethanolic solutions while filtering through a cotton plug. Final ethanolic concentration is 30%.

Maturation at room temperature during 3 hours. The ethanolic solution of C₂₅diynetrioxaamine becomes white opaque, forming a nanoemulsion.

### 6.3. Photopolymerization at 254 nm

Introduction of 5 ml samples of the previously formed nanoemulsion into 12 Petri dishes (35 mm in diameter)
Irradiation at 254 nm during 10 minutes in an UV-crosslinker (six 8W light tubes; Biolink BLX-E254 Vilbert Lourmat)

The polymerized solutions of C₂₅diynetrioxaamine turn dark-blue with large aggregates.

### 6.4. pH inversion and dissociation of aggregates of nanoemusion of C25diynetrioxaamine

Photopolymerized batches obtained previously are acidified by addition of hydrochloric acid 1N (50 µl per 5 ml sample; amounting to 0,3 mmoles in total) and heated in a sonication bath at 55°C for 1 hour.

The solutions of photopolymerized C₂₅diynetrioxaamine become intensely red coloured, while the larger aggregated dissociate into nanofibers. These red solutions contain the photopolymerized C₂₅diynetrioxaamine in nanofiber form.

These samples have been taken as such for biological evaluation.

SEQUENCE LISTING

<110>  Université de Strasbourg
       Centre National de la Recherche Scientifique

<120>  AMPHIPHILIC MONOMERS BASED NANOVECTORS AND THEIR USE FOR siRNA
       DELIVERY

<130>  SATC DIC

<160>  4

<170>  PatentIn version 3.5

<210>  1
<211>  23
<212>  DNA
<213>  Artificial Sequence

<220>
<223>  Synthetic Olginucleotide

<400>  1
cuuacgcuga guacuucgad tdt                                              23


<210>  2
<211>  23
<212>  DNA
<213>  Artificial Sequence

<220>
<223>  Synthetic Oligonucleotide

<400>  2
ucgaaguacu cagcguaagd tdt                                              23


<210>  3
<211>  23
<212>  DNA
<213>  Artificial Sequence

<220>
<223>  Synthetic oligonucleotide

<400>  3
cguacgcgga auacuucgad tdt                                              23


<210>  4
<211>  23
<212>  DNA
<213>  Artificial Sequence

<220>
<223>  Synthetic oligonucleotide

<400>  4
ucgaaguauu ccgcguacgd tdt                                              23

## Claims

1. Amphiphilic monomer of formula (I)
L₁-C-C-C-C-L₂-X-Z (I)
wherein
L₁ is CH₃(CH₂)ₘ, with m being an integer from 1 to 24,
L₂ is -CH₂(CH₂)ₙ-CONH-, with n being an integer from 1 to 24,
X is -(CH₂)ₜ-O-(CH₂CH₂O)ₚ(CH₂)_{t'}-NH-, with t and t' being independently from each other 2 or 3 and p being an integer from 2 to 500 and
Z is H or a positively charged group selected from
- natural or non natural amino acid residues,
- carboxylated polyamines residues, and
- peptides comprising from 2 to 5 amino acids, at least one of said amino-acids being an histidine residue,
and their physiologically acceptable salts.

2. Amphiphilic monomer of formula (I) according to claim 1 wherein Z is selected from:
- the following natural amino acid residues: 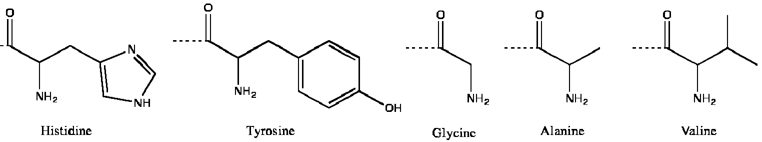
- non natural amino acid residues selected from the group comprising the enantiomers of the above mentioned natural amino acids and their beta and gamma analogues,
- a dipeptide comprising at least one histidine residue,
- a carboxyspermine residue of formula (1), 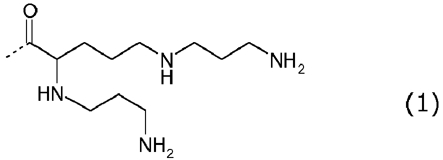
- a -COCH(NH2)(CH2)qA group with q being an integer from 1 to 5 and A being a hydrophilic head being chosen from imidazol-4-yl, imidazol-2-yl, imidazole-1-yl, phenol, 4-methylpiperazine-1-yl, N-substituted cycloamines and analogues thereof, and
their physiologically acceptable salts.

3. A polymerized micelle comprising a polymer obtainable by the photopolymerization of one monomer of formula (I) or of copolymers of monomers of formula (I) with the proviso that when there is only one monomer, then Z is not H.

4. A polymerized fiber comprising a polymer obtainable by the photopolymerization of one monomer of formula (I) or of copolymers of monomers of formula (I).

5. A transfection complex comprising a polymerized micelle according to claim 3 or a polymerized fiber according to claim 4 and at least one isolated nucleic acid.

6. A transfection complex according to claim 5 wherein the isolated nucleic acid is a small interfering RNA.

7. A pharmaceutical composition comprising a transfection complex according to any of claims 5 or 6 and a pharmaceutically acceptable excipient.

8. An in vitro transfection or delivery method comprising the step of introducing a transfection complex according to any of claims 5 or 6 into a host cell.

9. An in vitro transfection method according to claim 8 wherein said host cell is an eukaryotic cell.

10. An in vitro transfection method according to claim 9 wherein said eukaryotic cell is a mammalian cell selected.

11. An in vitro transfection method according to claim 10 wherein said mammalian cell is a human cell.

12. Process for obtaining polymerized micelles according to claim 3 or polymerized fibers according to claim 4, **characterized in that** it comprises the following steps:
- at least two amphiphilic momomers of formula (I), identical or different, are self-assembled into spherical micelles or fibers,
- the self-assembled spherical micelles or fibers are photopolymerized.
